# EUROPEAN PATENT SPECIFICATION

(11) **EP 2 999 358 B1**
(45) Date of publication and mention of the grant of the patent: **07.07.2021**
(21) Application number: 14800640.6
(22) Date of filing: 22.05.2014
(51) Int. Cl.: A23L 33/00, C07H 3/06, C07H 5/04, C07H 15/04, A61P 31/00

(54) **SYNTHETIC MIXTURE OF OLIGOSACCHARIDES FOR THE TREATING A MICROBIOTA OF A MAMMAL**
SYNTHETISCHE MISCHUNG AUS OLIGOSACCHARIDEN ZUR BEHANDLUNG EINER BIOZÖNOSE EINES SÄUGETIERS
MÉLANGE SYNTHÉTIQUE D'OLIGOSACCHARIDES POUR LE TRAITEMENT D'UN MICROBIOTE DE MAMMIFÈRE

(30) Priority: 22.05.2013 DK 201370275
(43) Date of publication of application: 30.03.2016
(73) Proprietor: Glycom A/S, 2970 Hørsholm (DK)
(72) Inventor: DEKANY, Gyula, Sinnamon Park, Queensland 4073 (AU)
(74) Representative: Inspicos P/S
(86) International application number: PCT/DK2014/050142
(87) International publication number: WO 2014/187464

(56) References cited:
- EP-A1- 2 143 341
- EP-A1- 2 465 508
- EP-A1- 2 522 232
- EP-B1- 3 389 404
- WO-A1-2012/007585
- WO-A1-2012/113405
- WO-A1-2012/156898
- WO-A1-2013/091660
- WO-A1-2013/190531
- WO-A2-2005/055944
- AU-A1- 2012 203 568
- GIBSON GLENN R ET AL: "Dietary modulation of the human colonic microbiota: updating the concept of prebiotics", NUTRITION RESEARCH REVIEWS,, vol. 17, no. 2, 1 December 2004 (2004-12-01), pages 259-275, XP009117209, DOI: 10.1079/NRR200479
- PIIRAINEN LAURA ET AL: "In school-aged children a combination of galacto-oligosaccharides and Lactobacillus GG increases bifidobacteria more than Lactobacillus GG on its own", ANNALS OF NUTRITION AND METABOLISM: EUROPEAN JOURNAL OF NUTRITION, METABOLIC DISEASES AND DIETETICS, S. KARGER AG, SWITZERLAND, vol. 52, no. 3, 1 July 2008 (2008-07-01), pages 204-208, XP009148647, ISSN: 0250-6807, DOI: 10.1159/000138125
- QIANG, X.; ET AL.: 'Health benefit application of functional oligosaccharides''.' CARBOHYDRATE POLYMERS vol. 77, 2009, pages 435 - 441, XP026119288

## Description

### FIELD OF THE INVENTION

The invention relates to the use of oligosaccharide blends for treating the microbiotas of humans suffering from the unhealthy effects of certain microbes.

### BACKGROUND OF THE INVENTION

Immediately after birth, humans become colonized by a range of microorganisms in different parts of the body, particularly on the skin, oronasopharyngeal area, gastrointestinal tract and urogenital tract, and during lifetime humans live in symbiosis with this diverse community of microbes. It is a known fact, that diseases und unhealthy conditions are associated with an altered composition of the microbiotas (disturbed balance) compared to that characteristic to the normal, healthy state. To explore how microbiome changes cause or prevent diseases and how a healthy microbiota can be restored are key step in understanding host -microbe interaction in health and disease and can be a potential for therapeutic manipulation of the microbiota (see e.g. Reid et al. Nat. Rev. Microbiol. 9, 27 (2011)).

The microbiota represents an important health asset essential for the development of the host, homeostasis and protection against pathogenic challenge, and carries a broad range of functions indispensable for the wellbeing such as aiding in nutrition and educating the immune system. The members of commensal microbiome are estimated to be around 100 trillion and its composition is characteristic for the healthy (as balanced community) and unhealthy (as unbalanced/disturbed community) status of the host. Studies about the taxonomic distribution of microorganisms in microbiotas in association with health and disease, partially within the frame of Human Microbiome Project (see Turnbaugh et al. Nature 449, 804 (2007)) have recently come into light (see for example Turnbaugh et al. Nature 444, 1027 (2006), Costello et al. Science 326, 1694 (2009), Zaura et al. BMC Microbiol. 9:259 (2009), Dominguez-Bello et al. Proc. Natl. Acad. Sci. USA 107, 11971 (2010), Grice et al. Proc. Natl. Acad. Sci. USA 107, 14799 (2010)).

In diverse places in and on the human body can be found microbiotas. For example in the human lower intestine and gut harbor a complex microbial community which utilizes dietary components that are non-digestible in the upper intestine. This diverse community of microorganisms, in a normal healthy individual, survives and functions in a balanced and dynamic equilibrium with its host (eubiosis) and with its microbial constituents. An imbalance in the microbiota community or its healthy functioning often results in health problems, as the microbiota contribute to many aspects of the host's growth and development. Thus, an imbalance in the gut microbiota can be associated with gastrointestinal tract disorders such as IBS, IBD, cholelithiasis and liver diseases as well as with complex diseases occurring in organs outside the gut, such as obesity, allergy, type 1 and type 2 diabetes and autism (see Sekirov et al. Physiol. Rev. 90, 859 (2010)).

Recently, Koeth et al. (Nature Medicine (2013) doi: 10.1038/nm.3145) revealed a new pathway in cardiovascular disease (CVD) pathogenesis in humans that involves unhealthy microbial metabolism of dietary L-carnitine. In this regard, it was shown that the gut microbiota of omnivorous subjects metabolizes L-carnitine and choline-containing lipids, found in red meat, to trimethylamine, which is further metabolized to trimethylamine-N-oxide (TMAO), a proatherogenic metabolite. It was demonstrated that omnivores produced more TMAO than vegetarians did, which correlates with the fact that the risk of atherosclerotic disease is lower in vegetarian individuals than in omnivores. Mice model studies showed that TMAO modulated cholesterol and sterol metabolism with the net effect of increasing atherosclerosis. Similar correlation was found at subjects with a diet rich in dietary lecithin (phosphatidylcholine found in eggs, liver, beef and pork) (Tang et al. N. Engl. J. Med. 368, 1575 (2013).

Ways have been sought, therefore, for reducing or preferably even eliminating diseases, such as atherosclerosis, that are associated with an imbalance in the microbiota and/or its proper functioning.

### SUMMARY OF THE INVENTION

In accordance with this invention, a synthetic mixture of oligosaccharides is provided for treating a microbiota of a mammal, advantageously a human, especially a human adult or senior, particularly an adult, to reduce or eliminate the activity and/or the proportion of a microbe in the microbiota that is associated with the development or maintenance of a disease or an unhealthy condition of the mammal, wherein the mixture comprises at least 6, particularly at least 8, more particularly at least 12, still more particularly at least 16, yet more particularly at least 20, even more particularly at least 24, quite particularly at least 30 oligosaccharides selected from the following:
- one or more GlcNAc-containing oligosaccharides, and/or
- one or more fucosylated oligosaccharides, and/or
- one or more sialylated oligosaccharides.

The synthetic mixture of oligosaccharides advantageously comprises at least one sialylated oligosaccharide and more advantageously comprises also a plurality of GlcNAc-containing oligosaccharides and/or fucosylated oligosaccharides and/or sialylated oligosaccharides. Yet more advantageously, the mixture of oligosaccharides comprises a plurality of GlcNAc-containing oligosaccharides and a plurality of fucosylated oligosaccharides and a plurality of sialylated oligosaccharides, and even more advantageously, this mixture contains:
- one or more mannose containing oligosaccharides, and/or
- one or more GalNAc-containing oligosaccharides, and/or
- one or more sulfated oligosaccharides.

In addition, the invention relates to a use of the aforesaid synthetic mixture of oligosaccharides for treating a microbiota of a mammal, advantageously a human, especially a human adult or senior, particularly an adult, to reduce or eliminate the activity and/or to reduce the relative abundance of a microbe in the unbalanced microbiota that is associated with the development or maintenance of a disease or an unhealthy condition of the mammal.

Moreover, the invention relates to a method for treating a microbiota of a mammal, advantageously a human, especially a human adult or senior, particularly an adult, to reduce or eliminate the activity and/or to reduce the relative abundance of a microbe in the unbalanced microbiota that is associated with the development or maintenance of a disease or an unhealthy condition of the mammal, comprising administering the aforesaid synthetic mixture of oligosaccharides to the mammal.

Furthermore, the invention relates to a pharmaceutical composition and a nutritional formulation comprising the aforesaid mixture of oligosaccharides for treating a microbiota of a mammal, preferably a human, especially a human adult or senior, particularly an adult, to reduce or eliminate the activity and/or to reduce the relative abundance of a microbe in the unbalanced microbiota that is associated with the development or maintenance of a disease or an unhealthy condition of the mammal.

### DETAILED DESCRIPTION OF THE INVENTION

In this invention, the term "fucosyl" preferably means the following L-fucopyranosyl group attached to a core oligosaccharide with an α-interglycosidic linkage:

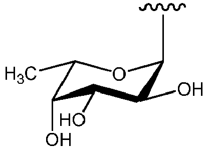

Herein, the term "N-acetyl-lactosaminyl group" preferably means the following the glycosyl residue of N-acetyl-lactosamine (LacNAc, Galpβ1-4GlcNAc) linked with a β-linkage:

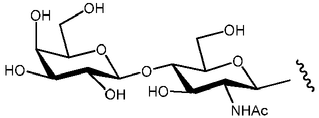

Also herein, the term "lacto-N-biosyl group" preferably means the following glycosyl residue of lacto-N-biose (LNB, Galpβ1-3GlcNAc) linked with a β-linkage:

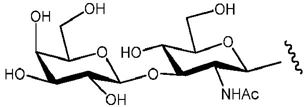

Further herein, the term "sialyl" preferably means the following glycosyl residue of sialic acid (N-acetyl-neuraminic acid, Neu5Ac) linked with an α-linkage:

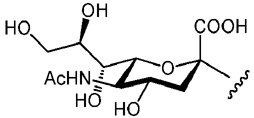

Still further herein, the term "glycosyl residue comprising one or more N-acetyl-lactosaminyl and/or one or more lacto-N-biosyl units" preferably means a linear or branched structure wherein such units are linked to each other by interglycosidic linkages.

Yet further herein, the term "GlcNAc-containing oligosaccharides" preferably means di-, tri-, tetra- and oligosaccharides of up to 12, preferably up to 10, more preferably up to 8 monomer units that are linked to each other by interglycosidic linkages and consist of N-acetyl-glucosamine and galactose and optionally glucose, representing a linear or a branched structure.

Also herein, the term "fucosylated oligosaccharides" preferably means GlcNAc-containing oligosaccharides defined above that are fucosylated and fucosylated lactose.

Further herein, the term "sialylated oligosaccharides" preferably means GlcNAc-containing oligosaccharides defined above that are sialylated, fucosylated oligosaccharides defined above that are sialylated and sialylated lactose.

Still further herein, the term "mannose containing oligosaccharides" preferably means di-, tri-, tetra- and oligosaccharides of up to 12, preferably up to 10, more preferably up to 8 monomer units that are linked to each other by interglycosidic linkages and consist of mannose and one or more naturally occurring monosaccharides of 5-9 carbon atoms selected from aldoses (e.g. glucose, galactose, ribose, arabinose, xylose, etc.), ketoses (e.g. fructose, sorbose, tagatose, etc.), deoxysugars (e.g. rhamnose, fucose, etc.), deoxy-aminosugars (e.g. N-acetyl-glycosamine, N-acetyl-mannosamine, N-acetyl-galactosamine, etc.), uronic acids and ketoaldonic acids (e.g. sialic acid).

Yet further herein, the term "GalNAc-containing oligosaccharides" preferably means di-, tri-, tetra- and oligosaccharides of up to 12, preferably up to 10, more preferably up to 8 monomer units that are linked to each other by interglycosidic linkages and consist of N-acetyl-galactosamine and one or more naturally occurring monosaccharides of 5-9 carbon atoms selected from aldoses (e.g. glucose, galactose, mannose, ribose, arabinose, xylose, etc.), ketoses (e.g. fructose, sorbose, tagatose, etc.), deoxysugars (e.g. rhamnose, fucose, etc.), deoxy-aminosugars (e.g. N-acetyl-glycosamine, N-acetyl-mannosamine, etc.), uronic acids and ketoaldonic acids (e.g. sialic acid).

Also herein, the term "sulfated oligosaccharides" preferably means di-, tri-, tetra- and oligosaccharides of up to 12, preferably up to 10, more preferably up to 8 monomer units that are linked to each other by interglycosidic linkages and consist of naturally occurring monosaccharides of 5-9 carbon atoms selected from aldoses (e.g. glucose, galactose, mannose, ribose, arabinose, xylose, etc.), ketoses (e.g. fructose, sorbose, tagatose, etc.), deoxysugars (e.g. rhamnose, fucose, etc.), deoxy-aminosugars (e.g. N-acetyl-glycosamine, N-acetyl-mannosamine, N-acetyl-galactosamine etc.), uronic acids and ketoaldonic acids (e.g. sialic acid), of which at least one (non-anomeric) OH-group is substituted with a sulfate ester.

Furthermore, the term "species" means any chemical compound, such as a toxin, that is produced by one or more microbes in the microbiota in the gut of a mammal and that is associated with, and can cause, a disease in the mammal.

The first aspect of the invention relates to a synthetic mixture of oligosaccharides that reduces or eliminates the activity of and/or diminishes the relative abundance of one or more microbes in the microbiota, particularly gut microbiota, that are associated with the development or maintenance of a disease or an unhealthy condition in a mammal, preferably a human, especially a human adult or senior, quite particularly an adult. In this regard, the mixture of oligosaccharides serves at least in part to modulate significantly the microbiota of the mammal to increase the amount and/or functioning of healthy microbes to reduce the amount and/or functioning of the disease-causing microbes. The mixture also serves, at least in part, to rebalance significantly the composition of the mammal's microbiota to increase the amount and/or functioning of healthy microbes and thereby reduce the amount and/or functioning of the disease-causing microbes. The mixture also serves, at least in part, to significantly restore a healthy composition of the mammal's microbiota and thereby to reduce the amount and/or functioning of the disease-causing microbes.

The synthetic mixture contains at least 6, preferably at least 8, more preferably at least 12, still more preferably at least 16, yet more preferably at least 20, even more preferably at least 24, quite preferably at least 30 oligosaccharides selected from the following:
- one or more GlcNAc-containing oligosaccharides, and/or
- one or more fucosylated oligosaccharides, and/or
- one or more sialylated oligosaccharides.

Preferably, the mixture of oligosaccharides comprises at least one sialylated oligosaccharide and more preferably also comprises a plurality of GlcNAc-containing oligosaccharides and/or fucosylated oligosaccharides and/or sialylated oligosaccharides. Yet more preferably, the mixture of oligosaccharides comprises a plurality of GlcNAc-containing oligosaccharides and a plurality of fucosylated oligosaccharides and a plurality of sialylated oligosaccharides, and even more preferably, this mixture contains:
- one or more mannose containing oligosaccharides, and/or
- one or more GalNAc-containing oligosaccharides, and/or
- one or more sulfated oligosaccharides.

With regard to different oligosaccharide groups contained in the mixture of oligosaccharides according to the first aspect of the invention, a GlcNAc-containing oligosaccharide is preferably a trisaccharide consisting of N-acetyl-glucosamine and lactose, especially lacto-N-triose (GlcNAcβ1-3Galβ1-4Glc).

Also preferably, the GlcNAc-containing oligosaccharide comprises an N-acetyl-lactosaminyl and/or a lacto-N-biosyl group. More preferably, any of the N-acetyl-lactosaminyl or lacto-N-biosyl groups can be substituted with a glycosyl residue comprising one or more N-acetyl-lactosaminyl and/or one or more lacto-N-biosyl moieties. Even more preferably, a lacto-N-biosyl group is not substituted further, and to an N-acetyl-lactosaminyl group another N-acetyl-lactosaminyl group can be attached with a 1-3 or 1-6 interglycosidic linkage (that is to the 3-OH or 6-OH group of the galactose in the N-acetyl-lactosamine), or to an N-acetyl-lactosaminyl group a lacto-N-biosyl group can be attached with a 1-3 interglycosidic linkage (that is to the 3-OH group of the galactose in the N-acetyl-lactosamine).

Also preferably, the GlcNAc-containing oligosaccharide comprising a N-acetyl-lactosaminyl and/or a lacto-N-biosyl group can further include a lactose unit, preferably at the reducing end. Within this group, compounds of formula **1** are especially preferred, 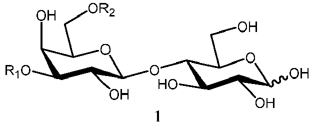
wherein R₁ is selected from H, N-acetyl-lactosaminyl and lacto-N-biosyl groups, wherein the N-acetyl lactosaminyl group can be substituted with a glycosyl residue consisting of one or more N-acetyl-lactosaminyl and/or one or more lacto-N-biosyl groups, and
R₂ is selected from H and N-acetyl-lactosaminyl group optionally substituted with a glycosyl residue consisting of one or more N-acetyl-lactosaminyl and/or one or more lacto-N-biosyl groups,
provided that R₁ and R₂ cannot be H simultaneously.

In a compound of formula **1**, preferably,
- the lacto-N-biosyl group is not substituted further and
- to the N-acetyl-lactosaminyl group in the glycosyl residue of R₁ another N-acetyl-lactosaminyl group can be attached with a 1-3 interglycosidic linkage,
- to the N-acetyl-lactosaminyl group in the glycosyl residue of R₁ a lacto-N-biosyl group can be attached with a 1-3 interglycosidic linkage,
- to the N-acetyl-lactosaminyl group in the glycosyl residue of R₂ another N-acetyl-lactosaminyl group can be attached with a 1-3 or a 1-6 interglycosidic linkage, and
- to the N-acetyl-lactosaminyl group in the glycosyl residue of R₂ a lacto-N-biosyl group can be attached with a 1-3 interglycosidic linkage.

These preferred compounds of formula **1** are core milk oligosaccharides that can be found in mammalian or human milk. The human core milk oligosaccharides containing N-acetyl-lactosamine and/or lacto-N-biose are listed in Table 1 below (see Urashima et al. Milk Oligosaccharides, Nova Biomedical Books, NY, 2011).

**Table 1.**

| No | Core name | Core structure |
|---|---|---|
| 1 | lacto-*N*-tetraose (LNT) | Galβ1-3GlcNAcβ1-3Galβ1-4Glc |
| 2 | lacto-*N*-neotetraose (LNnT) | Galβ1-4GlcNAcβ1-3Galβ1-4Glc |
| 3 | lacto-*N*-hexaose (LNH) | Galβ1-3GlcNAcβ1-3(Galβ1-4GlcNAcβ1-6)Galβ1-4Glc |
| 4 | lacto-*N*-neohexaose (LNnH) | Galβ1-4GlcNAcβ1-3(Galβ1-4GlcNAcβ1-6)Galβ1-4Glc |
| 5 | para-lacto-*N*-hexaose (para-LNH) | Galβ1-3GlcNAcβ1-3Galβ1-4GlcNAcβ1-3Galβ1-4Glc |
| 6 | para-lacto-*N*-neohexaose (para-LNnH) | Galβ1-4GlcNAcβ1-3Galβ1-4GlcNAcβ1-3Galβ1-4Glc |
| 7 | lacto-*N*-octaose (LNO) | Galβ1-3GlcNAcβ1-3(Galβ1-4GlcNAcβ1-3Galβ1-4GlcNAcβ1-6)Galβ1-4Glc |
| 8 | lacto-*N*-neooctaose (LNnO) | Galβ1-4GlcNAcβ1-3(Galβ1-3GlcNAcβ1-3Galβ1-4GlcNAcβ1-6)Galβ1-4Glc |
| 9 | iso-lacto-*N*-octaose (iso-LNO) | Galβ1-3GlcNAcβ1-3(Galβ1-3GlcNAcβ1-3Galβ1-4GlcNAcβ1-6)Galβ1-4Glc |
| 10 | para-lacto-*N*-octaose (para-LNO) | Galβ1-3GlcNAcβ1-3Galβ1-4GlcNAcβ1-3Galβ1-4GlcNAcβ1-3Galβ1-4Glc |
| 11 | lacto-*N*-neodecaose (LNnD) | Galβ1-3GlcNAcβ1-3[Galβ1-4GlcNAcβ1-3(Galβ1-4GlcNAcβ1-6)Galβ1-4GlcNAcβ1-6]Galβ1-4Glc |
| 12 | lacto-*N*-decaose (LND) | Galβ1-3GlcNAcβ1-3[Galβ1-3GlcNAcβ1-3(Galβ1-4GlcNAcβ1-6)Galβ1-4GlcNAcβ1-6]Galβ1-4Glc |

Also preferably, the GlcNAc-containing oligosaccharide is selected from the group consisting of N-acetyl-lactosamine (Galβ1-4GlcNAc), lacto-N-biose (Galβ1-3GlcNAc), lacto-N-triose (GlcNAcβ1-3Galβ1-4Glc), Galβ1-3GlcNAcβ1-3Gal, Galβ1-4GlcNAcβ1-3Gal, Galβ1-3GlcNAcβ1-3Galβ1-4GlcNAc, Galβ1-4GlcNAcβ1-3Galβ1-4GlcNAc, Galβ1-3GlcNAcβ1-3Galβ1-4GlcNAcβ1-3Galβ1-4GlcNAc, Galβ1-4GlcNAcβ1-3(Galβ1-4GlcNAcβ1-6)Galβ1-4GlcNAc, Galβ1-3GlcNAcβ1-3(Galβ1-4GlcNAcβ1-6)Galβ1-4GlcNAc, LNT, LNnT, LNH, LNnH, para-LNH and para-LNnH.

The fucosyl oligosaccharides in the mixture of the first aspect of the invention are GlcNAc-containing oligosaccharides defined above that are fucosylated. The fucose residue is attached to a galactose or an N-acetyl-glucosamine moiety. Favourably, a fucosyl residue can be attached to the galactose of a lacto-N-biosyl or a N-acetyl-lactosaminyl group with 1-2, 1-3, 1-4 or 1-6 interglycosidic linkage, preferably with 1-2 linkage, and/or to the N-acetyl-glucosamine of a lacto-N-biosyl group with 1-4 or 1-6 interglycosidic linkage, preferably with 1-4 linkage, and/or to the N-acetyl-glucosamine of a N-acetyl-lactosaminyl group with 1-3 or 1-6 interglycosidic linkage, preferably with 1-3 linkage. Additionally, the above fucosylated oligosaccharides can further include a lactose unit, preferably at the reducing end, forming compounds of formula **2** 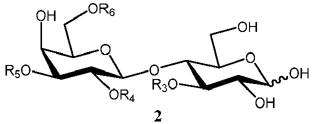
wherein R₃ is fucosyl or H,
R₄ is fucosyl or H,
R₅ is selected from H, N-acetyl-lactosaminyl and lacto-N-biosyl groups, wherein the N-acetyl lactosaminyl group can be substituted with a glycosyl residue comprising one or more N-acetyl-lactosaminyl and/or one or more lacto-N-biosyl groups; each of the N-acetyl-lactosaminyl and lacto-N-biosyl groups can be substituted with one or more fucosyl residue, R₆ is selected from H and N-acetyl-lactosaminyl groups optionally substituted with a glycosyl residue comprising one or more N-acetyl-lactosaminyl and/or one or more lacto-N-biosyl groups; each of the N-acetyl-lactosaminyl and lacto-N-biosyl groups can be substituted with one or more fucosyl residue,
provided that:
   R₃, R₄, R₅ and R₆ are not H in the same time, and
   if R₃ and R₄ are H then at least one of the R₅ and R₆ groups contains a fucosyl moiety.

The fucosylated oligosaccharides according to formula **2** are preferably those can be found as components in human milk (see Urashima et al. Milk Oligosaccharides, Nova Biomedical Books, NY, 2011) and some of them listed in Table 2.

**Table 2.**

| No | name | structure |
|---|---|---|
| 1 | LNFP I | Fucα1-2Galβ1-3GlcNAcβ1-3Ga1β1-4Glc |
| 2 | LNFP II | Galβ1-3(Fucα1-4)GlcNAcβ1-3Galβ1-4Glc |
| 3 | LNFP III | Galβ1-4(Fucα1-3)GlcNAcβ1-3Galβ1-4Glc |
| 4 | LNFP V | Galβ1-3GlcNAcβ1-3Galβ1-4(Fucα1-3)Glc |
| 5 | LNDFH I | Fucα1-2Galβ1-3(Fucα1-4)GlcNAcβ1-3Galβ1-4Glc |
| 6 | LNDFH II | Galβ1-3(Fucα1-4)GlcNAcβ1-3Galβ1-4(Fucα1-3)Glc |
| 7 | LNDFH III | Galβ1-4(Fucα1-3)GlcNAcβ1-3Galβ1-4(Fucα1-3)Glc |
| 8 | F-LNH I | Fucα1-2Galβ1-3GlcNAcβ1-3(Galβ1-4GlcNAcβ1-6)Galβ1-4Glc |
| 9 | F-LNH II | Galβ1-3GlcNAcβ1-3(Galβ1-4[Fucα1-3]GlcNAcβ1-6)Galβ1-4Glc |
| 10 | F-para-LNH I | Galβ1-3GlcNAcβ1-3Galβ1-4(Fucα1-3)GlcNAcβ1-3Galβ1-4Glc |
| 11 | F-para-LNH II | Galβ1-3(Fucα1-4)GlcNAcβ1-3Galβ1-4GlcNAcβ1-3Galβ1-4Glc |
| 12 | F-LNnH | Fucα1-{Galβ1-4GlcNAcβ1-3(Galβ1-4GlcNAcβ1-6)Galβ1-4Glc} [the position of the fucosyl residue has not elucidated yet] |
| 13 | DF-LNH I | Galβ1-3[Fucα1-4]GlcNAcβ1-3(Galβ1-4[Fucα1-3]GlcNAcβ1-6)Galβ1-4Glc |
| 14 | DF-LNH II | Fucα1-2Galβ1-3GlcNAcβ1-3(Galβ1-4[Fucα1-3]GlcNAcβ1-6)Galβ1-4Glc |
| 15 | DF-LNnH | Galβ1-4[Fucα1-3]GlcNAcβ1-3(Galβ1-4[Fucα1-3]GlcNAcβ1-6)Galβ1-4Glc |
| 16 | DF-para-LNH | Galβ1-3(Fucα1-4)GlcNAcβ1-3Galβ1-4(Fucα1-3)GlcNAcβ1-3Galβ1-4Glc |
| 17 | DF-para-LNnH | Galβ1-4(Fucα1-3)GlcNAcβ1-3Galβ1-4(Fucα1-3)GlcNAcβ1-3Galβ1-4Glc |

Furthermore, the fucosylated oligosaccharides can also be fucosylated lactoses, among which 2'-FL (Fucα1-2Galβ1-4Glc), 3-FL (Galβ1-4[Fucα1-3]Glc) and DFL (Fucα1-2Galβ1-4[Fucα1-3]Glc) are preferred.

More preferably, the fucosylated oligosaccharides are selected from the group consisting of Galβ1-3(Fucα1-4)GlcNAc (Le^{a}), Fucα1-2Galβ1-3(Fucα1-4)GlcNAc (Le^{b}), Galβ1-4(Fucα1-3)GlcNAc (Le^{x}), Fucα1-2Galβ1-4(Fucα1-3)GlcNAc (Le^{y}), 2'-FL, 3-FL, DFL, LNFP I, LNFP II, LNFP III, LNFP V, F-LNnH, DF-LNH I, DF-LNH II, DF-LNH I, DF-para-LNH and DF-para-LNnH.

The sialyl oligosaccharides in the mixture of the first aspect of the invention are GlcNAc-containing oligosaccharides defined above that are sialylated. Preferably, the sialyl residue is attached to the galactose of the lacto-N-biosyl group with 2-3 interglycosidic linkage and/or to the N-acetyl-glucosamine of the lacto-N-biosyl or the N-acetyl-lactosaminyl group with 2-6 interglycosidic linkage and/or to the galactose of the N-acetyl-lactosaminyl group with 2-3 or 2-6 interglycosidic linkage. Furthermore, the above sialylated oligosaccharides can further include a lactose unit, preferably at the reducing end, giving compounds of formula **3** 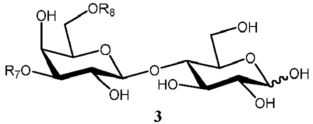
wherein R₇ is selected from H, N-acetyl-lactosaminyl and lacto-N-biosyl groups, wherein the N-acetyl lactosaminyl group can be substituted with a glycosyl residue comprising one or more N-acetyl-lactosaminyl and/or one or more lacto-N-biosyl groups; each of the N-acetyl-lactosaminyl and lacto-N-biosyl groups can be substituted with one or more sialyl residue,
R₈ is selected from H and N-acetyl-lactosaminyl groups optionally substituted with a glycosyl residue comprising one or more N-acetyl-lactosaminyl and/or one or more lacto-N-biosyl groups; each of the N-acetyl-lactosaminyl and lacto-N-biosyl groups can be substituted with one or more sialyl residue,
provided that:
   R₇ and R₈ are not H in the same time, and
   at least one sialyl moiety is present.

The sialylated oligosaccharides according to formula **3** are preferably those can be found as components in human milk (see Urashima et al. Milk Oligosaccharides, Nova Biomedical Books, NY, 2011) and some of them listed in Table 3.

**Table 3.**

| No | name | structure |
|---|---|---|
| 1 | LST a | Neu5Acα2-3Galβ1-3GlcNAcβ1-3Galβ1-4Glc |
| 2 | LST b | Galβ1-3(Neu5Acα2-6)GlcNAcβ1-3Galβ1-4Glc |
| 3 | LST c | Neu5Acα2-6Galβ1-4GlcNAcβ1-3Galβ1-4Glc |
| 4 | S-LNH | Galβ1-3GlcNAcβ1-3(Neu5Acα2-6Galβ1-4GlcNAcβ1-6)Galβ1-4Glc |
| 5 | S-LNnH I | Galβ1-4GlcNAcβ1-3(Neu5Acα2-6Galβ1-4GlcNAcβ1-6)Galβ1-4Glc |
| 6 | S-LNnH II | Neu5Acα2-6Galβ1-4GlcNAcβ1-3(Galβ1-4GlcNAcβ1-6)Galβ1-4Glc |
| 7 | DS-LNT | Neu5Acα2-3Gaβ1-3(Neu5Aca2-6)GlcNAcβ1-3Galβ1-4Glc |
| 8 | DS-LNH I | Neu5Acα2-3Galβ1-3GlcNAcβ1-3(Neu5Acα2-6Galβ1-4GlcNAcβ1-6)Galβ1-4Glc |
| 9 | DS-LNH II | Neu5Acα2-3Galβ1-3[Neu5Aca2-6]GlcNAcβ1-3(Galβ1-4GlcNAcβ1-6)Galβ1-4Glc |
| 10 | DS-LNnH | Neu5Acα2-6Galβ1-4GlcNAcβ1-3(Neu5Acα2-6Galβ1-4GlcNAcβ1-6)Galβ1-4Glc |
| 11 | TS-LNH | Neu5Acα2-3Galβ1-3[Neu5Acα2-6]GlcNAcβ1-3(Neu5Acα2-6Galβ1-4GlcNAcβ1-6)Galβ1-4Glc |

Additionally, the sialyl oligosaccharides can be also fucosylated GlcNAc-containing oligosaccharides defined above that are sialylated, thus giving sialylated and fucosylated GlcNAc-containing glycans. Preferably, the sialyl residue is attached to the galactose of the lacto-N-biosyl group with 2-3 interglycosidic linkage and/or to the N-acetyl-glucosamine of the lacto-N-biosyl group with 2-6 interglycosidic linkage and/or to the galactose of the N-acetyl-lactosaminyl group with 2-3 or 2-6 interglycosidic linkage in those oligosaccharides. More preferably, only not fucosylated galactose residue can be sialylated. Among the sialylated and fucosylated GlcNAc-containing glycans defined above the ones found in human milk are preferred (see Urashima et al. Milk Oligosaccharides, Nova Biomedical Books, NY, 2011) and some of them listed in Table 4.

**Table 4.**

| No | name | structure |
|---|---|---|
| 1 | F-LST a | Neu5Acα2-3Galβ1-3(Fucα1-4)GlcNAcβ1-3Galβ1-4Glc |
| 2 | F-LST b | Fucα1-2Galβ1-3(Neu5Acα2-6)GlcNAcβ1-3Galβ1-4Glc |
| 3 | F-LST c | Neu5Acα2-6Galβ1-4GlcNAcβ1-3Galβ1-4(Fucα1-3)Glc |
| 4 | FS-LNH | Fucα1-2Galβ1-3GlcNAcβ1-3(Neu5Acα2-6Galβ1-4GlcNAcβ1-6)Galβ1-4Glc |
| 5 | FS-LNH I | Galβ1-3[Neu5Acα2-6]GlcNAcβ1-3(Galβ1-4[Fucal-3]GlcNAcβ1-6)Galβ1-4Glc |
| 6 | FS-LNH II | Neu5Acα2-3Galβ1-3GlcNAcβ1-3(Galβ1-4[Fucα1-3]GlcNAcβ1-6)Galβ1-4Glc |
| 7 | FS-LNH III | Galβ1-3[Fucα1-4]GlcNAcβ1-3(Neu5Acα2-6Galβ1-4GlcNAcβ1-6)Galβ1-4Glc |
| 8 | FS-LNH IV | Neu5Acα2-3Galβ1-3[Fuca1-4]GlcNAcβ1-3(Galβ1-4GlcNAcβ1-6)Galβ1-4Glc |
| 9 | FS-LNnH I | Neu5Acα2-6Galβ1-4GlcNAcβ1-3(Galβ1-4[Fucα1-3]GlcNAcβ1-6)Galβ1-4Glc |
| 10 | FDS-LNT I | Neu5Acα2-3Galβ1-3(Neu5Acα2-6)[Fucα1-4]GlcNAcβ1-3Galβ1-4Glc |
| 11 | FDS-LNT II | Neu5Acα2-3Galβ1-3(Neu5Acα2-6)GlcNAcβ1-3Galβ1-4[Fucα1-3]Glc |

Furthermore, the sialyl oligosaccharides can also be lactose and fucosylated lactoses that are sialylated, among which 3'-SL (Neu5Acα2-3Galβ1-4Glc), 6'-SL (Neu5Acα2-6Galβ1-4Glc) and FSL (Neu5Acα2-3Galβ1-4[Fucα1-3]Glc) are preferred.

Additionally, the sialyl oligosaccharide can be oligo- or polysialic acid wherein the sialic acid monomers are coupled to each other via α2-8 and/or α2-9 interglycosidic linkages.

Yet further, the sialylated GlcNAc-containing oligosaccharides defined above, sialylated and fucosylated GlcNAc-containing oligosaccharides defined above, sialylated lactoses defined above, and sialylated and fucosylated lactoses as defined above can be further sialylated at their sialyl residues, wherein the substituent sialyl moiety/moieties is/are coupled with α2-8 and/or α2-9 interglycosidic linkages.

More preferably, the sialylated oligosaccharides are selected from the group consisting of Neu5Acα2-3Galβ1-3(Fucl-4)GlcNAc (SLe^{a}), Neu5Acα2-3Galβ1-3GlcNAc(Le^{c}), Neu5Acα2-3Galβ1-4GlcNAc, Neu5Acα2-3Galβ1-4(Fucα1-3)GlcNAc (SLe^{x}), 3'-SL, 6'-SL, FSL, F-LST a, F-LST b, F-LST c, LST a, LST b, LST c and DS-LNT.

In a synthetic mixture of oligosaccharides comprising mannose containing oligosaccharides, the mannose containing oligosaccharides are preferably N-glycans, or derivatives and analogs thereof. N-glycans are mannose containing oligosaccharides that are covalently linked to proteins at asparagine via an N-glycosidic bond (see: Stanley et al.: N-glycans, in: Essentials of glycobiology (Varki et al. eds.), 2nd edition, Cold Spring Harbour, NY (2009)). Thus in this regard, the mannose containing oligosaccharides preferably comprise a Manβ1-4GlcNAc sequence, more preferably Manα1-6(Manα1-3)Manβ1-4GlcNAc, even more preferably this sequence is coupled to a GlcNAc to form Manα1-6(Manα1-3)Manβ1-4GlcNAcβ1-4GlcNAc. Furthermore, to the terminal mannose residue(s)more mannose moieties can be attached comprising Manα1-2Man and/or Manα1-3Man and/or Manα1-6Man residues (oligomannoses), or to the terminal mannose residue(s) N-acetyl-lactosamine and/or lacto-N-biose moieties, preferably N-acetyl-lactosamine, optionally substituted with sialyl and/or fucosyl moieties, can be attached to form antennary structures, or in the Manα1-6(Manα1-3)Man(31-4GlcNAcβ1-4GlcNAc core only mannose residues are attached to the Manα1-6 arm and N-acetyl-lactosamine containing glycosyl residue optionally substituted with sialyl and/or fucosyl moieties to the Manα1-3 arm. In addition, a mannose containing oligosaccharides can be a mannosyl glucose or an oligosaccharide containing a mannosyl glucose disaccharide moiety. Furthermore, a mannose containing oligosaccharide can be a mannosyl lactose or an oligosaccharide containing a mannosyl lactose trisaccharide moiety. In a mixture of oligosaccharides comprising GalNAc-containing oligosaccharides, the GalNAc-containing oligosaccharides are preferably GlcNAc-containing oligosaccharides as defined above, or fucosylated GlcNAc-containing oligosaccharides as defined above, or sialylated GlcNAc-containing oligosaccharides as defined above, or sialylated and fucosylated GlcNAc-containing oligosaccharides as defined above, wherein at least one GlcNAc is replaced with GalNAc. Especially preferred when the GalNAc moiety is present in the reducing end, even more preferably the GalNAc-containing oligosaccharide is Galβ1-3GalNAc or an oligosaccharide containing Galβ1-3GalNAc in the reducing end. In addition, a GalNAc-containing oligosaccharide can be an N-acetyl-lactosamine or lacto-N-biose containing oligosaccharide defined above to which a GalNAc residue is attached, preferably an N-acetyl-lactosamine containing oligosaccharide to which a GalNAc residue is attached, more preferably the GalNAc is coupled to the galactosyl moiety of the N-acetyl-lactosamine residue, even more preferably via a β1-3 linkage.

The sulfated oligosaccharides in the synthetic mixture of the first aspect of the invention are di-, tri-, tetra- and oligosaccharides of up to 12, preferably up to 10, more preferably up to 8 monomer units that are linked to each other by interglycosidic linkages and consist of naturally occurring monosaccharides of 5-9 carbon atoms selected from aldoses (e.g. glucose, galactose, mannose, ribose, arabinose, xylose, etc.), ketoses (e.g. fructose, sorbose, tagatose, etc.), deoxysugars (e.g. rhamnose, fucose, etc.), deoxy-aminosugars (e.g. glucosamine, N-acetyl-glycosamine, mannosamine, N-acetyl-mannosamine, galactosamine, N-acetyl-galactosamine etc.), uronic acids and ketoaldonic acids (e.g. sialic acid), of which at least one (non-anomeric) OH-group or at least one free amino group (if present) is substituted with a sulfate (as ester or amide, respectively). Preferably, sulfated oligosaccharides are sialylated oligosaccharides as defined above, wherein one or more sialyl moieties are replaced by sulfate esters.

In accordance with the first aspect of the invention, synthetic oligosaccharide mixtures as defined above can reduce or preferably eliminate the activity of one or more microbes in the microbiota that are associated with the development or maintenance of a disease or an unhealthy condition in a mammal. Also, the oligosaccharide mixtures can reduce or preferably eliminate the activity of one or more microbes in the microbiota that can produce species or precursor(s) thereof associated with the development or maintenance of a disease or an unhealthy condition in a mammal.

The synthetic oligosaccharide mixtures of this invention can be used to treat a disease or an unhealthy condition in a wide variety of mammals. The mammals can be human or non-human, the latter group including primates (e.g. monkeys, chimpanzees, etc.), companion animals (e.g. dogs, cats, equine, rodent pets, etc.), farm animals (e.g. goats, sheep, swine, bovine, etc.), laboratory animals (e.g. mice, rats, etc.), and wild and zoo animals (e.g. wolves, bears, deer, etc.). The mammals are preferably human.

The synthetic oligosaccharide mixtures of this invention can be useful for treating a variety of microbiota (or microbial flora) inhabiting different regions or organs of a mammal. Such microbiota particularly include the microbial communities of the skin, oronasopharyngeal area (oral cavity), gastrointestinal tract or gut (stomach, small intestine [duodenum, jejunum, ileum], large intestine [colon]) and urogenital tract. Such microbiota are preferably gut microbiota, more preferably colon microbiota.

Heretofore, significant modulation, restoration and/or rebalancing of a mammalian microbiota have never been achieved. Antibiotic treatments do not selectively target unhealthy microbes as they suppress substantially the entire microbiota. Functional foods containing probiotics can be used to a very limited extent for this purpose, but at present they are limited to providing only Lactobacilli and Bifidobacteria.

In this invention, the term "modulation of a microbiota" preferably means altering the composition or overall state of a microbiota, so that the concentration and/or unhealthy activity of unhealthy microbes in the microbiota is significantly reduced by increasing the concentration and/or healthy activity of healthy microbes in the microbiota. Also herein, the term "restoration of a microbiota" preferably means altering the composition or overall state of a microbiota to restore the composition of the mammal's microbiota to a previous healthier state, so that the concentration and/or healthy activity of healthy microbes in the microbiota is significantly increased relative to the concentration and/or unhealthy activity of unhealthy microbes in the microbiota. Also herein, the term "rebalancing of a microbiota" preferably means altering the composition or overall state of a microbiota, so that the relative concentration of healthy and unhealthy microbes in the microbiota is significantly altered to resemble that of a healthy microbiota. The selective modulation is believed to be due to the high structural diversity of the oligosaccharides mixture.

The microbiota modulation, restoration and/or rebalancing of this invention with its synthetic oligosaccharide mixture reduces, or preferably eliminates, the relative abundance, and thereby one or more undesirable activities, of one or more unhealthy or disease related microbes, either commensal or exogenous, associated with the development or maintenance of an unhealthy condition or a disease by:
- promoting the colonization of healthy or not disease related microbes in the microbiota, thus shifting the microbiota composition towards a healthier balance;
- inhibiting the colonization of the unhealthy or disease related microbes in the microbiota; and/or
- reducing or eliminating the undesirable activities of the unhealthy or disease related microbes in the microbiota.

The undesirable activities of the unhealthy or disease related microbes, that are to be reduced, can involve a metabolic production and secretion of toxins, endotoxins, oligosaccharides, glycolipids, short chain fatty acids and/or low molecular weight signalling molecules that are associated with the development or maintenance of a disease or an unhealthy condition in a mammal.

The undesirable activities of the unhealthy or disease related microbes, that are to be reduced, can also involve a metabolic production and secretion of cell-surface anchored molecules that are immobilized on the cell surface and thereby affect intracellular interactions such as cell-toxin interactions, cell-hormone interactions, cell-enzyme interactions and/or cell-antibody interactions, associated with the development or maintenance of a disease or an unhealthy condition in a mammal.

The diseases and unhealthy conditions, associated with unhealthy or disease related microbes, are not confined to the organs where such microbes are situated in mammals. In this regard, the unhealthy or disease related microbes can have an impact on a wide range of diseases and conditions such as infections (e.g., airway infections, gut infections, hospital-acquired infections), diabetes (both type 1 and 2), IBD, IBS, obesity, cardiovascular diseases, immunological disorders (mainly at elderly people), autism, dysphagia, maternal malnutrition, acne, allergy, cancer metastasis, digestive trauma (due to e.g., chemotherapy, antibiotic treatment or surgery), wound healing, etc.

In carrying out this invention, the undesirable activities of the unhealthy or disease related microbes, to be treated, involve a metabolic production of a species, e.g., a toxin, or a precursor thereof that is associated with the development or maintenance of a disease or an unhealthy condition.

Specialized groups of microbes of the gut microbiota are believed to participate in digesting different dietary components and metabolically to produce toxins, compounds or precursors thereof responsible for unhealthy effects. In this regard, a preferred embodiment of this invention provides a carefully selected synthetic oligosaccharide blend that reduces, suppresses or inhibits the metabolic production of toxins, compounds or precursors thereof in the gut microbiota, therefore a targeted gut microbiota modulation, restoration or rebalancing can be achieved. This microbiota restoration implies the curtailment of the growth of the toxin or toxin precursor producing bacterial strains present in the unbalanced microbiota which thus drives the microbiota towards eubiosis.

Also preferably, the disease associated with the dysbiosis of the gut microbiota described above is a cardiovascular disease, such as heart disease (ischemic heart disease, coronary heart disease, cardiomyopathy, hypertensitive heart disease, heart failure, cardiac dysrhythmia, inflammatory heart disease, rheumatic heart disease), cerebrovascular disease (such as stroke), arteriosclerotic vascular disease (such as atherosclerosis), more preferably atherosclerosis.

In this regard, a species or a precursor thereof that is associated with the development or maintenance of a cardiovascular disease, preferably atherosclerosis, in a mammal, preferably in a human, is a toxin or a toxin precursor.

More preferably, the toxin associated with the development or maintenance of atherosclerosis is trimethylamine-N-oxide (TMAO). TMAO inhibits reverse cholesterol transport (RCT), the cholesterol elimination from the body, therefore causes or maintains atherosclerosis. TMAO is produced from trimethyl-amine (TMA) in the liver in an oxidation procedure by flavin monooxygenases, therefore TMA is considered to be the precursor of TMAO. TMA is a metabolic product of certain microbes of the gut microbiota. Bacterial taxa that can be associated with the metabolic production of TMA are Deferribacteres, Tenericutes and certain Bacteroidetes (Koeth et al. Nature Medicine (2013) doi:10.1038/nm.3145). Food ingredients or xenobiotics that can serve carbon and energy source for microbes producing TMA are organic trimethylammonium derivatives, preferably selected from carnitine, choline, phosphatidyl cholines (lecithin) and betaine.

In another embodiment, the microbiota treated selectively is a skin microbiota. Thus a carefully selected oligosaccharide blend is provided that reduces the relative abundance of the microbe(s), associated with the development or maintenance of a skin disease, Preferably, the skin disease is a chronic wound that is a complication of the diabetes. More preferably, the microbe of which relative abundance to be reduced is Staphylococcus (see Grice et al. Proc. Natl. Acad. Sci. USA 107, 14799 (2010)).

The individual components in the mixture of oligosaccharides according to the first aspect of the invention are known carbohydrates and can be prepared by usual manners in a chemical way, by enzymatic means or by microbial production. As examples, using chemistry LNnT can be made as described in WO 2011/100980 and WO 2013/044928, LNT can be synthesized as disclosed in WO 2012/155916 and WO 2013/044928, mixture of LNT and LNnT can be done according to PCT/DK2012/050502, LNnH and para-LNnH can be made as shown in WO 2013/044928, 2'-FL can be produced as described in WO 2010/115934 and WO 2010/115935, 3-FL can be furnished according to PCT/DK2013/050078, 6'-SL and salts thereof can be manufactured by WO 2010/100979, sialylated oligosaccharides can be produced as outlined in 2012/113404 and mixture of human milk oligosaccharides can be prepared as disclosed in WO 2012/113405. As examples of enzymatic production, sialylated oligosaccharides can be made as described in WO2012/007588, fucosylated oligosaccharides can be obtained as specified in WO 2012/127410, and advantageously diversified blends of human milk oligosaccharides can be furnished according to WO 2012/156897 and WO 2012/156898. With regard to biotechnological methods, WO 01/04341 and WO 2007/101862 describe the production core human milk oligosaccharides optionally substituted by fucose or sialic acid using genetically modified *E. coli.*

The second aspect of the invention relates to the use of a synthetic mixture of oligosaccharides, for reducing or eliminating the activity and/or diminishing the relative abundance of one or more microbes in the microbiota that are associated with the development or maintenance of a disease or an unhealthy condition in a mammal, preferably a human, especially a human adult or senior, particularly an adult. In this regard, the use of the mixture of oligosaccharides serves at least in part to rebalance the gut microbiota of the mammal to increase the amount and/or functioning of healthy microbes to reduce the amount and/or functioning of the disease-causing microbes. The use of the mixture also serves, at least in part, to restore the composition of the mammal's microbiota to a previous healthier state, so as to increase the amount and/or functioning of healthy microbes to reduce the amount and/or functioning of the disease-causing microbes. The use of mixture also serves, at least in part, to modulate the composition of the mammal's microbiota to reduce the amount and/or functioning of the disease-causing microbes. The mixture contains at least 6, particularly at least 8, more particularly at least 12, still more particularly at least 16, yet more particularly at least 20, even more particularly at least 24, quite particularly at least 30 oligosaccharides selected from the following:
- one or more GlcNAc-containing oligosaccharides, and/or
- one or more fucosylated oligosaccharides, and/or
- one or more sialylated oligosaccharides.

Preferably, the mixture of oligosaccharides comprises at least one sialylated oligosaccharide and more preferably also comprises a plurality of GlcNAc-containing oligosaccharides and/or fucosylated oligosaccharides and/or sialylated oligosaccharides. More advantageously, the mixture of oligosaccharides comprises a plurality of GlcNAc-containing oligosaccharides and a plurality of fucosylated oligosaccharides and a plurality of sialylated oligosaccharides, and even more advantageously, this mixture contains:
- one or more mannose containing oligosaccharides, and/or
- one or more GalNAc-containing oligosaccharides, and/or
- one or more sulfated oligosaccharides.

The third aspect of the invention relates to a method of treating an imbalanced gut microbiota of a mammal, preferably a human, for reducing or eliminating the activity and or diminishing the relative abundance of one or more microbes in the microbiota that are associated with the development or maintenance of a disease in a mammal, preferably a human, especially an adult or senior, particularly an adult. In this regard, the treatment serves at least in part to rebalance the gut microbiota of the mammal to increase the amount and/or functioning of healthy microbes to reduce the amount and/or functioning of the disease-causing microbes. The treatment also serves, at least in part, to rebalance the composition of the mammal's microbiota to increase the amount and/or functioning of healthy microbes to reduce the amount and/or functioning of the disease-causing microbes. The treatment also serves, at least in part, to modulate the composition of the mammal's microbiota to reduce the amount and/or functioning of the disease-causing microbes. The method comprises administering a synthetic mixture of oligosaccharides that contains at least 6, particularly at least 8, more particularly at least 12, still more particularly at least 16, yet more particularly at least 20, even more particularly at least 24, quite particularly at least 30 oligosaccharides selected from the following:
- one or more GlcNAc-containing oligosaccharides, and/or
- one or more fucosylated oligosaccharides, and/or
- one or more sialylated oligosaccharides.

Preferably, the mixture of oligosaccharides comprises at least one sialylated oligosaccharide and more preferably also comprises a plurality of GlcNAc-containing oligosaccharides and/or fucosylated oligosaccharides and/or sialylated oligosaccharides. More advantageously, the mixture of oligosaccharides comprises a plurality of GlcNAc-containing oligosaccharides and a plurality of fucosylated oligosaccharides and a plurality of sialylated oligosaccharides, and even more advantageously, this mixture contains:
- one or more mannose containing oligosaccharides, and/or
- one or more GalNAc-containing oligosaccharides, and/or
- one or more sulfated oligosaccharides.

In the fourth aspect of the invention, a pharmaceutical composition is provided for treating infants, children, adults and/or seniors and particularly subjects having specialized needs (e.g., suffering from metabolic disorders), comprising the synthetic oligosaccharide mixture according to the first aspect. The mixture of oligosaccharides can be added to a pharmaceutically acceptable carriers such as conventional additives, adjuvants, excipients and diluents (water, gelatine, talc, sugars, starch, gum arabic, vegetable gums, vegetable oils, polyalkylene glycols, flavouring agents, preservatives, stabilizers, emulsifying agents, lubricants, colorants, fillers, wetting agents, etc.). Suitable carriers are described in the most recent edition of Remington's Pharmaceutical Sciences, a standard reference text in the field. When the mixture of oligosaccharides is added to the pharmaceutically acceptable carriers a dosage in the form of for example, but not limited to tablets, powders, granules, suspensions, emulsions, infusions, capsules, injections, liquids, elixirs, extracts and tincture can be made, which especially useful when the gut microbiota is treated. For skin microbiota treatment, a solution, gel (e.g. water-based), cream, emollient, foam, ointment, lotion, shampoo or spray can be used. The most suitable formulas for the treatment of the oral cavity can be solid formulations such as lozenges, lollipops, troches, dragees, chewable gums, solid candies, granular solids such as powders, chewable tablets or pills, orally dispersable tablets or pills, orally dissolvable tablets, pills or capsules, and the like, as well as liquid or semi-solid formulations such as solutions, suspensions, pastes, creams, lotions, and emulsions. To the above formulas, if needed, probiotics, e.g. lacto bacteria, *Bifidobacterium* species, prebiotics such as fructooligosaccharides and galactooligosaccharides, proteins from casein, soy-bean, whey or skim milk, carbohydrates such as lactose, saccharose, maltodextrin, starch or mixtures thereof, lipids (e.g. palm olein, sunflower oil, safflower oil) and vitamins and minerals essential in a daily diet can also be further added.

Pharmaceutical compositions comprising the oligosaccharide mixture according to the first aspect can be manufactured by means of any usual manner known in the art, e.g. described in the most recent edition of Remington's Pharmaceutical Sciences, a standard reference text in the field.

The fifth aspect of the invention is a nutritional formulation, such as food, drink or feed, containing the synthetic oligosaccharide mixture of the first aspect and conventional edible micronutrients, vitamins and minerals. The amounts of such ingredients can vary depending on whether the consumable product is intended for use with infants, children, adults, seniors or subjects having specialized needs (e.g., suffering from metabolic disorders). Micronutrients include for example edible oils, fats or fatty acids (such as coconut oil, soy-bean oil, monoglycerides, diglycerides, palm olein, sunflower oil, fish oil, linoleic acid, linolenic acid etc.), carbohydrates (such as glucose, fructose, sucrose, maltodextrin, starch, hydrolyzed cornstarch, etc.) and proteins from casein, soy-bean, whey or skim milk, or hydrolysates of these proteins, but protein from other source (either intact or hydrolysed) can be used as well. Vitamins can be chosen such as vitamin A, B1, B2, B5, B6, B12, C, D, E, H, K, folic acid, inositol and nicotinic acid. The nutritional formulation can contain the following minerals and trace elements: Ca, P, K, Na, Cl, Mg, Mn, Fe, Cu, Zn, Se, Cr or I. Furthermore additional probiotics can be added, e.g. lacto bacteria, *Bifidobacterium* species, prebiotics such as fructooligosaccharides and galactooligosaccharides, proteins from casein, soy-bean, whey or skim milk, carbohydrates such as lactose, saccharose, maltodextrin, starch or mixtures thereof, lipids (e.g. palm olein, sunflower oil, safflower oil) and vitamins and minerals essential in a daily diet can also be further added.

In a preferred embodiment, the nutritional formulation comprising the oligosaccharide mixture according to the first aspect can be a food supplement. Such a food supplement preferably contains ingredients as defined for nutritional food above, e.g. vitamins, minerals, trace elements and other micronutrients, etc. The food supplement can be for example in the form of tablets, capsules, pastilles or a liquid. The supplement can contain conventional additives selected from but not limited to binders, coatings, emulsifiers, solubilising agents, encapsulating agents, film forming agents, adsorbents, carriers, fillers, dispersing agents, wetting agents, jellifying agents, gel forming agents, etc.

In another preferred embodiment, the nutritional formulation comprising the oligosaccharide mixture according to the first aspect can be digestive health functional food as the administration of the mixture of oligosaccharides according to the present invention provides a beneficial effect on digestive health. Digestive health functional food is preferably a processed food used with intention to enhance and preserve digestive health by utilizing the mixture of oligosaccharides according to the present invention as physiologically functional ingredients or components in forms of tablet, capsule, powder, etc. Different terms such as dietary supplement, nutraceutical, designed food, health product can also be used to refer to digestive health functional food. The nutritional formulation comprising the oligosaccharide mixture according to the first aspect can be prepared in any usual manner. For example, it can be prepared by admixing micronutrient components in appropriate proportions. Then the vitamins and minerals are added, but to avoid thermal degradation or decomposition heat sensitive vitamins can be added after homogenization. Lipophilic vitamins can be dissolved in the fat source before mixing. A liquid mixture is formed using water, whose temperature is preferably about between 50-80 °C to help dissolution or dispersal of the ingredients. Oligosaccharide mixture according to the first aspect can be added at this stage. The resulting mixture is then homogenized by flash heating to about 80-150 °C by means of steam injection, heat exchanger or autoclave. This thermal treatment reduces significantly the bacterial loads as well. The hot mixture is then cooled rapidly to about 60-80 °C. If needed, further homogenization can be carried out at this temperature under high pressure of about 2-30 MPa. After cooling heat sensitive constituents can be added at this stage, and the pH and the content of the solids are conveniently adjusted. The resulting mixture is then dried by conventional method such as spray drying or freeze drying to powder. Probiotics can be added at this point by dry-mixing.

### EXAMPLE

Synthetic mixtures of oligosaccharides are provided for treating a microbiota of a mammal, preferably a human, especially a human adult or senior, particularly an adult, to reduce or eliminate the activity and/or the proportion of a microbe in the microbiota that is associated with the development or maintenance of a disease or an unhealthy condition of the mammal. The mixture comprises at least 6, particularly at least 8, more particularly at least 12, still more particularly at least 16, yet more particularly at least 20, even more particularly at least 24 oligosaccharides selected from the following oligosaccharides nos. 1-28:

| | **Short Name** | **Full Name** | | **Short Name** | **Full Name** |
|---|---|---|---|---|---|
| 1 | 2'-FL | 2'-O-fucosyllactose | 15 | para-LNnH | para-lacto-N-neohexaose |
| 2 | LNFP I | lacto-N-fucopentaose I | 16 | S-LNnH I | sialyllacto-N-neohexaose I |

| | **Short Name** | **Full Name** | | **Short Name** | **Full Name** |
|---|---|---|---|---|---|
| 3 | LNT | lacto-N-tetraose | 17 | S-LNnH II | sialyllacto-N-neohexaose II |
| 4 | 3-FL | 3-O-fucosyllactose | 18 | DS-LNnH | disialyllacto-N-neohexaose |
| 5 | 6'-SL | 6'-O-sialyllactose | 19 | F-LNnH | fucosyllacto-N-neohexaose |
| 6 | DFL | Lactodifucotetraose / Difucosyllactose | 20 | DF-LNnH | difucosyllacto-N-neohexaose |
| 7 | LNFP II | lacto-N-fucopentaose II | 21 | LST d | 3"'-O-sialyllacto-N-neotetraose |
| 8 | LST c | 6"'-O-sialyllacto-N-neotetraose | 22 | S-LNnH I | sialyllacto-N-neohexaose I |
| 9 | LNnT | lacto-N-neotetraose | 23 | S-LNnH II | sialyllacto-N-neohexaose II |
| 10 | LNFP III | lacto-N-fucopentaose III | 24 | DS-LNnH | disialyllacto-N-neohexaose |
| 11 | 3'-SL | 3'-O-sialyllactose | 25 | DS-LNT | disialyllacto-N-tetraose |
| 12 | LNnH | lacto-N-neohexaose | 26 | LNB | lacto-N-biose |
| 13 | FSL | 3'-O-sialyl-3-O-fucosyllactose | 27 | LacNAc | N-acetyl lactosamine |
| 14 | LST a | 3"'-O-sialyllacto-N-tetraose | 28 | lacto-N-triose | lacto-N-triose |

## Claims

1. A synthetic mixture of oligosaccharides for use in treating a microbiota of a mammal to reduce or eliminate the activity and/or the proportion of a microbe in the microbiota that is associated with the development or maintenance of a disease or an unhealthy condition of the mammal, wherein the mixture comprises at least 6 oligosaccharides selected from the following:
- one or more GlcNAc-containing oligosaccharides, and/or
- one or more fucosylated oligosaccharides, and/or
- one or more sialylated oligosaccharides,
wherein the mammal is a human adult or senior.

2. The mixture for the use according to claim 1, wherein the mixture comprises at least 8, particularly at least 12, more particularly at least 16, still more particularly at least 20, yet more particularly at least 24, quite particularly at least 30 oligosaccharides

3. The mixture for the use according to claim 1 or 2, wherein the mixture comprises at least one sialylated oligosaccharide, preferably wherein the mixture comprises at least a plurality of GlcNAc-containing oligosaccharides, fucosylated oligosaccharides and/or sialylated oligosaccharides, in particular wherein the mixture comprises a plurality of GlcNAc-containing oligosaccharides, a plurality of fucosylated oligosaccharides and a plurality of sialylated oligosaccharides.

4. The mixture for the use according to any one of claims 1-3, wherein each GlcNAc-containing oligosaccharide is selected from LacNAc, lacto-N-biose, lacto-N-triose, LNT, LNnT, LNH, LNnH, para-LNH and para-LNnH.

5. The mixture for the use according to any one of claims 1-4, wherein each fucosylated oligosaccharide is selected from 2'-FL, 3-FL, DFL, LNFP I, LNFP II, LNFP III, LNFP V, F-LNnH, DF-LNH I, DF-LNH II, , DF-para-LNH and DF-para-LNnH.

6. The mixture for the use according to any one of claims 1-5, wherein each sialylated oligosaccharide is selected from 3'-SL, 6'-SL, FSL, F-LST a, F-LST b, F-LST c, LST a, LST b, LST c and DS-LNT.

7. The mixture for use according to any one of claims 1-6 for treating a microbiota, wherein the activity of a microbe involves a metabolic production of a species, particularly a toxin, or a precursor thereof that is associated with the development or maintenance of a disease of the mammal.

8. The mixture for the use according to any one of claims 1-7 wherein the microbiota is a gut microbiota.

9. The mixture for the use according to claim 8, wherein the disease is a cardiovascular disease, gastrointestinal tract disorder, obesity, diabetes or autism.

10. The mixture for the use according to claim 9, wherein the disease is a cardiovascular disease, particularly atherosclerosis.

11. The mixture for the use according to claim 10, wherein the disease is atherosclerosis and the toxin precursor is trimethyl amine.

12. The mixture for the use according to any one of claims 1-7 for treating a microbiota, wherein the microbiota is a skin microbiota.

13. The mixture for the use according to claim 12, wherein the unhealthy condition is a chronic wound caused by diabetes.

14. A pharmaceutical composition for the use as defined in any of the precedent claims comprising a synthetic mixture of oligosaccharides as defined in any one of claims 1-6.

15. A nutritional formulation for the use as defined in any of the precedent claims comprising a synthetic mixture of oligosaccharides as defined in any one of claims 1-6.

## Patentansprüche

1. Eine Synthetische Mischung aus Oligosacchariden zur Verwendung bei der Behandlung der Mikrobiota eines Säugetiers, um die Aktivität und/oder den Anteil einer Mikrobe in der Mikrobiota zu reduzieren oder zu eliminieren, die mit der Entwicklung oder Aufrechterhaltung einer Erkrankung oder eines ungesunden Zustands des Säugers verbunden ist, wobei die Mischung mindestens 6 Oligosaccharide umfasst, die aus den Folgenden ausgewählt sind:
- einem oder mehreren GlcNAc-haltigen Oligosacchariden und/oder
- einem oder mehreren fucosylierten Oligosacchariden und/oder
- einem oder mehreren sialylierten Oligosacchariden, wobei das Säugetier ein menschlicher Erwachsener oder Senior ist.

2. Die Mischung zur Verwendung nach Anspruch 1, wobei die Mischung mindestens 8, bevorzugt mindestens 12, mehr bevorzugt mindestens 16, stärker bevorzugt mindestens 20, noch stärker bevorzugt mindestens 24, ganz besonders bevorzugt mindestens 30 Oligosaccharide umfasst.

3. Die Mischung zur Verwendung nach Anspruch 1 oder 2, wobei die Mischung mindestens ein sialyliertes Oligosaccharid umfasst, wobei die Mischung vorzugsweise mindestens eine Vielzahl von GlcNAc-haltigen Oligosacchariden, fucosylierten Oligosacchariden und/oder sialylierten Oligosacchariden umfasst, wobei die Mischung insbesondere eine Vielzahl von GlcNAc-haltigen Oligosacchariden, eine Vielzahl von fucosylierten Oligosacchariden und eine Vielzahl von sialylierten Oligosacchariden umfasst.

4. Die Mischung zur Verwendung nach einem der Ansprüche 1-3, wobei jedes GlcNAc enthaltende Oligosaccharid aus LacNAc, Lacto-N-Biose, Lacto-N-Triose, LNT, LNnT, LNH, LNnH, para-LNH und para-LNnH ausgewählt ist.

5. Die Mischung zur Verwendung nach einem der Ansprüche 1-4, wobei jedes fucosylierte Oligosaccharid aus 2'-FL, 3-FL, DFL, LNFP I, LNFP II, LNFP III, LNFP V, F-LNnH, DF-LNH I, DF-LNH II, DF-para-LNH und DF-para-LNnH ausgewählt ist.

6. Die Mischung zur Verwendung nach einem der Ansprüche 1-5, wobei jedes sialylierte Oligosaccharid aus 3'-SL, 6'-SL, FSL, F-LST a, F-LST b, F-LST c, LST a, LST b, LST c und DS-LNT ausgewählt ist.

7. Die Mischung zur Verwendung nach einem der Ansprüche 1-6 zur Behandlung einer Mikrobiota, wobei die Aktivität einer Mikrobe eine metabolische Produktion einer Spezies, insbesondere eines Toxins, oder eines Vorläufers davon , die mit der Entwicklung oder Aufrechterhaltung einer Erkrankung des Säugers verbunden ist, umfasst.

8. Die Mischung zur Verwendung nach einem der Ansprüche 1-7, wobei die Mikrobiota eine Darm-Mikrobiota ist.

9. Die Mischung zur Verwendung nach Anspruch 8, wobei die Erkrankung eine Herz-Kreislauf-Erkrankung, eine Störung des Magen-Darm-Trakts, Fettleibigkeit, Diabetes oder Autismus ist.

10. Die Mischung zur Verwendung nach Anspruch 9, wobei die Erkrankung eine kardiovaskuläre Erkrankung, insbesondere Atherosklerose, ist.

11. Die Mischung zur Verwendung nach Anspruch 10, wobei die Erkrankung Atherosklerose ist und der Toxinvorläufer Trimethylamin ist.

12. Die Mischung zur Verwendung nach einem der Ansprüche 1-7 zur Behandlung einer Mikrobiota, wobei die Mikrobiota eine Hautmikrobiota ist.

13. Die Mischung zur Verwendung nach Anspruch 12, wobei der ungesunde Zustand eine chronische, durch Diabetes verursachte Wunde ist.

14. Eine Pharmazeutische Zusammensetzung zur Verwendung nach einem der vorhergehenden Ansprüche, die eine synthetische Mischung aus Oligosacchariden nach einem der Ansprüche 1-6 umfasst.

15. Eine Nährstoffformulierung zur Verwendung nach einem der vorhergehenden Ansprüche, die eine synthetische Mischung aus Oligosacchariden nach einem der Ansprüche 1-6 umfasst.

## Revendications

1. Un mélange synthétique d'oligosaccharides pour son utilisation dans le traitement d'un microbiote d'un mammifère pour réduire ou éliminer l'activité et/ou la proportion d'un microbe dans le microbiote qui est associé au développement ou à la persistance d'une maladie ou d'un état pathologique du mammifère, dans lequel le mélange comprend au moins 6 oligosaccharides sélectionnés parmi les suivants :
- un ou plusieurs oligosaccharides contenant un residue GlcNAc, et/ou
- un ou plusieurs oligosaccharides fucosylés, et/ou
- un ou plusieurs oligosaccharides sialylés,
dans lequel le mammifère est un adulte ou sénior humain.

2. Le mélange pour son utilisation selon la revendication 1, dans lequel le mélange comprend au moins 8, particulièrement au moins 12, plus particulièrement au moins 16, encore plus particulièrement au moins 20, toujours plus particulièrement au moins 24, vraiment particulièrement au moins 30 oligosaccharides.

3. Le mélange pour son utilisation selon la revendication 1 ou 2, dans lequel le mélange comprend au moins un oligosaccharide sialylé, de préférence dans lequel le mélange comprend au moins une pluralité d'oligosaccharides contenant un residue GlcNAc, d'oligosaccharides fucosylés, et/ou d'oligosaccharides sialylés, en particulier dans lequel le mélange comprend une pluralité d'oligosaccharides contenant un residue GlcNAc, une pluralité d'oligosaccharides fucosylés et une pluralité d'oligosaccharides sialylés.

4. Le mélange pour son utilisation selon l'une quelconque des revendications 1 à 3, dans lequel chaque oligosaccharide contenant un residue GlcNAc est sélectionné parmi la LacNAc, le lacto-N-biose, le lacto-N-triose, le LNT, le LNnT, le LNH, le LNnH, le para-LNH et le para-LNnH.

5. Le mélange pour son utilisation selon l'une quelconque des revendications 1 à 4, dans lequel chaque oligosaccharide fucosylé est sélectionné parmi le 2'-FL, le 3-FL, le DFL, le LNFP I, le LNFP II, le LNFP III, le LNFP V, le F-LNnH, le DF-LNH I, le DF-LNH II, le DF-para-LNH et le DF-para-LNnH.

6. Le mélange pour son utilisation selon l'une quelconque des revendications 1 à 5, dans lequel chaque oligosaccharide sialylé est sélectionné parmi le 3'-SL, le 6'-SL, le FSL, le F-LST a, le F-LST b, le F-LST c, le LST a, le LST b, le LST c et le DS-LNT.

7. Le mélange pour son utilisation selon l'une quelconque des revendications 1 à 6 pour le traitement d'un microbiote, dans lequel l'activité d'un microbe implique une production métabolique d'une espèce, en particulier d'une toxine, ou d'un précurseur de celle-ci qui est associé(e) au développement ou à la persistance d'une maladie du mammifère.

8. Le mélange pour son utilisation selon l'une quelconque des revendications 1 à 7 dans laquelle le microbiote est un microbiote intestinal.

9. Le mélange pour son utilisation selon la revendication 8, dans lequel la maladie est une maladie cardiovasculaire, un trouble du tractus gastrointestinal, l'obésité, le diabète ou l'autisme.

10. Le mélange pour son utilisation selon la revendication 9, dans lequel la maladie est une maladie cardiovasculaire, notamment l'athérosclérose.

11. Le mélange pour son utilisation selon la revendication 10, dans lequel la maladie est l'athérosclérose et le précurseur de la toxine est la triméthylamine.

12. Le mélange pour son utilisation selon l'une quelconque des revendications 1 à 7 pour le traitement d'un microbiote, dans lequel le microbiote est un microbiote cutané.

13. Le mélange pour son utilisation selon la revendication 12, dans lequel la condition pathologique est une plaie chronique provoquée par le diabète.

14. Une composition pharmaceutique pour son utilisation selon l'une quelconque des revendications précédentes comprenant un mélange synthétique d'oligosaccharides tel que défini dans l'une quelconque des revendications 1 à 6.

15. Une formulation nutritionnelle pour son utilisation selon l'une quelconque des revendications précédentes comprenant un mélange synthétique d'oligosaccharides tel que défini dans l'une quelconque des revendications 1 à 6.
